# EUROPEAN PATENT APPLICATION

(11) **EP 4 769 257 A1**
(43) Date of publication of application: **01.07.2026**
(21) Application number: 25172899.4
(22) Date of filing: 28.04.2025
(51) Int. Cl.: G06Q 10/04, G06Q 10/0631, G01N 33/00

(54) **SPATIAL UPSCALING FOR MONITORING REPORTING AND VERIFICATION OF GREENHOUSE GAS FLUX IN A GEOGRAPHICALLY BOUNDED AREA**

(30) Priority: 31.12.2024 US 202419006411
(71) Applicant: Hyphen Global AG, 4058 Basel (CH)
(72) Inventor: Austin, Miles, 4058 Basel (CH); Dilley, Frank Brian, 1296 Coppet (CH); Gonzalez Avilés, Ruben Octavio, 8004 Zu rich (CH)
(74) Representative: Bringer IP

(57) **Abstract**

Spatial upscaling for greenhouse gas flux quantification over a sparsely sensed geographically bounded region includes configuring a data structure to include a number of uniform spatial units, each representing a location within a geographically bounded area. Greenhouse gas flux data are received from different sensors disposed within the area and are stored in the corresponding uniform spatial units of the data structure. For each uniform spatial unit not associated with a disposed sensor, environmental context data for that location are selected. A flux model is queried with the retrieved environmental context data to estimate the greenhouse gas flux for that spatial unit. A token is then generated from the data structure indicative of a total mass of greenhouse gas emitted/sequestered across all locations within the larger geographically bounded area.

## Description

### BACKGROUND OF THE INVENTION

### Field of the Invention

The present invention relates to the technical field of greenhouse gas flux measurement over a large geographically bounded area.

### Description of the Related Art

The purpose of measuring greenhouse gases is to understand their role in climate change and to mitigate its dire effects. Greenhouse gases, such as carbon dioxide (CO₂), methane (CH₄), and nitrous oxide (N₂O), trap heat in the Earth's atmosphere. Atmospheric concentrations of these three gases in particular are increasing due to human activity, increasing the greenhouse effect. By measuring these gases, scientists can track changes in their levels over time and assess their impact on the environment and human society.

Greenhouse gases are monitored worldwide via a dedicated network of surface-based atmospheric monitoring stations and satellite-based sensors. However, since surface-based samples are taken infrequently and from a limited number of locations, and satellites and research projects provide data only discontinuously, capturing the full extent of greenhouse gas dynamics across different regions and over time faces coverage, accuracy and consistency limitations. Improving the data on the exchanges of greenhouse gases between the earth's surface and the atmosphere (fluxes) for different ecosystems is currently a critical scientific undertaking.

One emerging method to combat the greenhouse effect is through carbon credits, which are awarded by quantifying the results of greenhouse gas emissions reduction and sequestration activities-such as storing CO₂ in oceans, forests, or underground. Each credit represents one ton of CO₂ or equivalent in other greenhouse gases reduced or removed from the atmosphere. By creating an economic value for emissions reductions and removals, carbon credits incentivize individuals, organizations and governments to invest in climate-friendly initiatives.

However, the popularity of carbon credits faces challenges due to conflicting and inaccurate data available to the public on atmospheric greenhouse gases, undermining confidence in their credibility and effectiveness as a climate change mitigation tool. Therefore, accurate data are fundamental for a sustainable future, where well-informed decisions and collective action are pivotal in addressing the severe impacts of greenhouse gases and climate change. In particular, there is a need to improve the accuracy of greenhouse gas flux quantification for specific geographically bounded areas to accurately reflect emissions reductions and removals achieved through the implementation of individual carbon projects.

### BRIEF SUMMARY OF THE INVENTION

Embodiments of the present invention address technical deficiencies of the art in respect to greenhouse gas flux quantification. To that end, embodiments of the present invention provide for a novel and non-obvious spatial upscaling method for greenhouse gas flux quantification over a sparsely sensed geographically bounded region. Embodiments of the present invention also provide for a novel and non-obvious computing device adapted to perform the foregoing method. Finally, embodiments of the present invention provide for a novel and non-obvious data processing system incorporating the foregoing device in order to perform the foregoing method.

In one embodiment of the invention, a spatial upscaling method for greenhouse gas flux quantification over a sparsely sensed geographically bounded region is provided. The method includes both defining a geographically bounded area in a data structure in memory of a host computing platform, and also configuring the data structure to include a discrete number of uniform spatial units, with each of the units representing a specific location within the geographically bounded area. The method additionally includes receiving greenhouse gas flux data from different sensors disposed within the geographically bounded area and storing the flux data in one or more of the corresponding uniform spatial units of the data structure.

In one aspect of the embodiment, the method also additionally includes receiving environmental context data for each location within the geographically bounded area and storing the environmental context data in the corresponding one of the uniform spatial units of the data structure. The method then includes the training of a flux model to identify the flux values associated with different aspects of the environmental context within the geographically bounded area, based on the association between the flux values at locations with disposed sensors and the different aspects of the environmental context stored in the uniform spatial units of the data structure for those same locations. The flux model training identifies systematic, complex and non-linear associations between environmental context data and measured fluxes, the latter derived from the different sensors disposed within the geographically bounded area.

Thereafter, for each one of the uniform spatial units in the data structure not associated with a disposed one of the different sensors, the environmental context data for that location is selected. The flux model, previously trained to identify the flux values associated with different aspects of the environmental context within the geographically bounded area, is then queried with the retrieved environmental context data in order to estimate the gas flux at that location. More specifically, the flux model predicts a flux value for each location within the geographically bounded area not associated with a disposed one of the sensors.

The predictive flux value obtained from the model is reflective of the environmental context data associated with known flux values from comparable known locations possessing similar environmental characteristics within the geographically bounded area. The predictive data for gas flux is assigned to a portion of the data structure associated with the corresponding one of the uniform spatial units not associated with a disposed one of the sensors. Finally, an emissions or removals token is generated from the data structure which is indicative of a total mass of greenhouse gas emitted or sequestered across all locations within the geographically bounded area.

Aspects of the embodiment can include variations which may be included in the broad embodiment alone or in any combination with one another as follows:
- Any portion of the gas flux data computed to fall outside a threshold range relative to a running average of gas flux data for each disposed sensor may be discarded from consideration.
- The sensors each include a greenhouse gas flux sensor measuring a concentration of greenhouse gas in the atmosphere and a directionality of flow of the greenhouse gas.
- The environmental context data include at least one of humidity, temperature, wind speed and direction and vegetation.
- The flux model training identifies systematic, complex and non-linear associations between environmental context data and measured fluxes.
- The spatial upscaling method includes using a weighted combination of measured fluxes and fluxes predicted by the model in assigning a flux value to each uniform spatial unit such that, for uniform spatial units corresponding to locations closer to a disposed one of the sensors, the measured flux values from the sensor are most highly weighted whereas, as distance from a disposed one of the sensors increases, the weighting accorded to the measured flux values from that sensor assigned to the uniform spatial units decreases, until the flux values become almost entirely predicted.
- The estimates from the flux model may be compared with measured flux values from locations with disposed sensors whose measured values were not used to train the model, in which case the differences between the predicted and measured fluxes at those locations are computed and used to correct the flux model estimates for all unmonitored locations.

In another embodiment of the invention, a data processing system is adapted for greenhouse gas flux quantification over a sparsely sensed geographically bounded region. The system includes a host computing platform of one or more computers, each with memory and one or more processing units including one or more processing cores. The system also includes multiple, different sensors disposed within a geographically bounded area. Each of the sensors is communicatively coupled to a network interface of the host computing platform. As such, the system yet further includes a data structure stored in the memory which defines the geographically bounded area. Even further, the system includes a flux model. The flux model, upon receiving environmental and contextual input data for a source location of the geographically bounded area, estimates a greenhouse gas flux value for a target location, which can be different than the source location and be remote from or adjacent to the source location, or which even can be the same as the source location.

Importantly, the system includes a spatial upscaling module. The spatial upscaling module includes computer program instructions which are enabled during the execution of the instructions in the memory of one or more of the processing units of the host computing platform to perform a spatial upscaling method for greenhouse gas flux quantification over a sparsely sensed geographically bounded region. The system includes a configuration of the data structure to include a discrete number of uniform spatial units, with each of the units representing a specific location within the geographically bounded area. Instructions in the memory of one or more of the processing units of the host computing platform store greenhouse gas flux data from the different sensors in one or more of the corresponding uniform spatial units of the data structure.

In one aspect of the embodiment, instructions in the memory of one or more of the processing units of the host computing platform retrieve greenhouse gas flux data from sensors disposed within the geographically bounded area and store it in the uniform spatial units of the data structure corresponding to locations with disposed sensors. The instructions also retrieve environmental context data from a cloud data service for each location within the geographically bounded area and store the environmental context data in each corresponding uniform spatial unit of the data structure. Instructions in the memory of one or more of the processing units of the host computing platform then train a flux model to identify the flux values associated with different aspects of the environmental context within the geographically bounded area, based on systematic, complex and non-linear associations between the environmental context data and flux values stored in the uniform spatial units of the data structure corresponding to disposed sensors.

Then, for each corresponding one of the uniform spatial units in the data structure not associated with a disposed one of the different sensors, the instructions select the corresponding environmental context data for that location. The instructions supply the flux model with the retrieved environmental context data. The flux model executed according to the instructions in the memory of one or more of the processing units of the host computing platform then estimates the gas flux at the location in the geographically bounded area corresponding to the one of the uniform spatial units in the data structure not associated with a disposed one of the sensors.

In this way, the flux model predicts a flux value for locations within the geographically bounded area not associated with a disposed one of the sensors. The flux values predicted by the model are reflective of the environmental context data associated with known flux values from comparable known locations possessing similar environmental characteristics within the geographically bounded area. The instructions assign the predictive data for gas flux to a portion of the data structure associated with the corresponding one of the uniform spatial units not associated with a disposed one of the sensors.

In assigning a flux value to each uniform spatial unit, the spatial upscaling instructions include using a weighted combination of measured fluxes and fluxes predicted by the model. For uniform spatial units corresponding to locations closer to a disposed one of the sensors, the measured flux values from the sensor are most highly weighted. As distance from a disposed one of the sensors increases, the weighting accorded to the measured flux values from that sensor assigned to the uniform spatial units decreases, until the flux values become almost entirely predicted. The instructions may also compare the flux values estimated by the model with measured flux values from locations associated with disposed sensors whose measured values were not used to train the model, in which case the instructions compute the differences between the estimated and measured fluxes at those locations and use the computed differences to correct the flux model estimates for all unmonitored locations not associated with one of the disposed sensors. Finally, the instructions generate an emissions or removals token from the data structure indicative of a total mass of greenhouse gas emitted or sequestered across all locations within the geographically bounded area.

In this way, the technical deficiencies of estimating greenhouse gas fluxes over time for a large geographically bounded area with only limited actual coverage of the geographically bounded area by disposed sensors - owing to a limited quantity, position and range of sensors - can be overcome, owing to training and use of the flux model to predict accurate greenhouse gas flux values for locations within the geographically bounded area outside of the actual coverage of the sensors.

Additional aspects of the invention will be set forth in part in the description which follows, and in part will be obvious from the description, or may be learned by practice of the invention. The aspects of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE SEVERAL VIEWS OF THE DRAWINGS

The accompanying drawings, which are incorporated in and constitute part of this specification, illustrate embodiments of the invention and together with the description, serve to explain the principles of the invention. The embodiments illustrated herein are presently preferred, it being understood, however, that the invention is not limited to the precise arrangements and instrumentalities shown, wherein:
Figure 1 is a pictorial illustration reflecting different aspects of a process of spatial upscaling for greenhouse gas flux quantification over a sparsely sensed geographically bounded region;
Figure 2 is a block diagram depicting a data processing system adapted to perform one of the aspects of the process of Figure 1; and,
Figure 3 is a flow chart illustrating one of the aspects of the process of Figure 1.

### DETAILED DESCRIPTION OF THE INVENTION

Embodiments of the invention provide a process for implementing a spatial upscaling method for greenhouse gas flux quantification over a sparsely sensed geographically bounded region. In accordance with an embodiment of the invention, a spatial upscaler partitions a geographically bounded area into cells, some of which include physically disposed surface sensor fixtures so as to monitor, at different time intervals, environmental observables including earthen measurements, atmospheric measurements including greenhouse gases, and meteorological measures. Others of the cells lack directly observed sensor data. Additional environmental observables are available for all cells within the bounded geographic area, however. For the monitored cells, greenhouse gas fluxes are measured from observed data provided by the physically disposed sensors.

To estimate flux values for unmonitored cells, at each time interval during a time period of greenhouse gas flux computation, a flux value is predicted for each of the unmonitored cells by a flux model, previously trained to identify the flux values associated with different aspects of the environmental context within the geographically bounded area, drawing upon both physically disposed sensor and cloud service observables data. In assigning a flux value to each cell, the spatial upscaling includes using a weighted combination of measured fluxes from the physically disposed sensors and fluxes predicted by the model. For cells corresponding to locations closer to a physically disposed sensors, the measured flux values from the sensor are most highly weighted.

As distance from a physically disposed sensor increases, the weighting accorded to the measured flux values from that sensor assigned to the cells decreases, until the flux values become almost entirely predicted. The estimates from the flux model may also be compared with measured flux values from cells with physically disposed sensors whose measured values were not used to train the model. In that case, the differences between the predicted and measured fluxes for those cells are computed and used to correct the flux model estimates for all cells lacking physically disposed sensors. In this way, a token can be minted for the time period encapsulating a computed greenhouse gas flux for the entirety of the geographically bounded area.

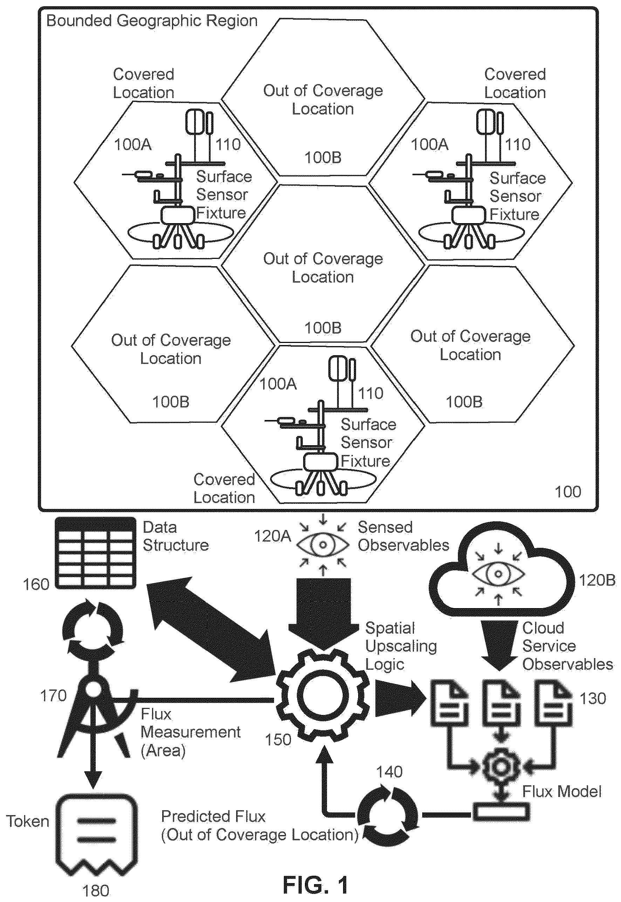

In illustration of one aspect of the embodiment, Figure 1 pictorially shows a process for implementing a spatial upscaling method for greenhouse gas flux quantification over a sparsely sensed geographically bounded region. As shown in Figure 1, spatial upscaling logic 150 defines a bounded geographical area 100 in a data structure 160 and partitions the bounded geographical area 100 into different cells 100A, 100B. Surface sensor fixtures 110 are disposed within a subset of monitored cells 100A amongst the different cells 100A, 100B while a remaining subset of unmonitored cells 100B amongst the different cells 100A, 100B lack the disposition of a corresponding surface sensor fixture 110.

Surface sensor fixtures 110 in the monitored cells 100A measure sensed observables 120A and, thereby, a greenhouse gas flux for the monitored cells 100A. In addition, cloud service observables 120B are also received for the monitored cells 100A which are associated with the greenhouse gas fluxes for the monitored cells 100A. As for each of the unmonitored cells 100B, for each corresponding one of the unmonitored cells 100B, cloud service observables 120B for the unmonitored cell 100B are provided to a flux model 130, previously trained to identify the flux values associated with various aspects of the environmental context within the geographically bounded area.

The flux model 130 returns a predicted greenhouse gas flux 140 for the corresponding one of the unmonitored cells 100B, derived from the association found for cells 100A between the observables 120A from the surface sensor fixtures 110, including the greenhouse gas fluxes, and the cloud service observables 120B. The spatial upscaling logic 150 then combines all greenhouse gas flux measurements and predicted flux values for the all cells 100A, 100B in order to compute a greenhouse gas flux measurement 170 for the bounded geographic area 100. More specifically, the flux model 130 can include a gradient boosting decision tree (GBDT) adapted for the regression analysis of a dynamically specified data set, in this instance, the greenhouse gas flux and other sensed observables 120A of the monitored cells 100A along with cloud service observables 120B for all cells 100A, 100B within the bounded geographic area 100.

In assigning a flux value to each cell 100A, 100B, the spatial upscaling logic 150 includes using a weighted combination of measured fluxes and fluxes predicted by the flux model 130. For cells corresponding to locations closer to a surface sensor fixture 110, the measured flux values from the sensor are most highly weighted. As distance from surface sensor fixture 110 in any of the different cells 100A, 100B increases, the weighting accorded to the measured flux values from that sensor assigned to any cell decreases, until the flux values become almost entirely predicted. The spatial upscaling logic 150 in consequence can mint a token 180 incorporating the total greenhouse gas flux quantity per unit time 170 for the bounded geographic area 100.

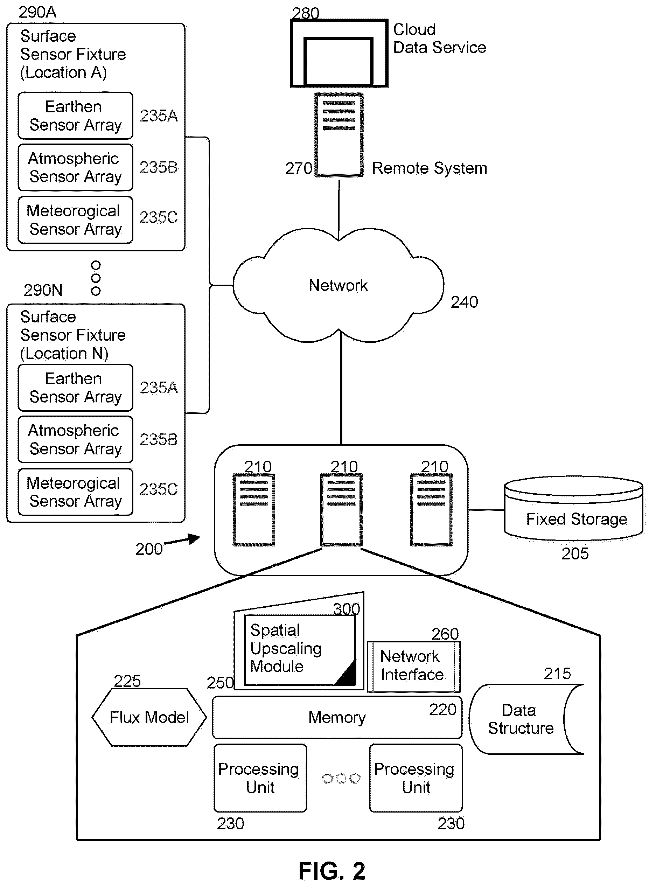

Aspects of the process described in connection with Figure 1 can be implemented within a data processing system. In further illustration, Figure 2 schematically shows a data processing system adapted to perform spatial upscaling for greenhouse gas flux quantification over a sparsely sensed geographically bounded region. In the data processing system illustrated in Figure 2, a host computing platform 200 is provided. The host computing platform 200 includes one or more computers 210, each with memory 220 and one or more processing units 230. The computers 210 of the host computing platform (only a single computer is shown, for the purpose of illustrative simplicity) can be co-located with one another and in communication with one another over a local area network or over a data communications bus. A network interface 260 further is provided to facilitate data communications between the host computing platform 200 and other computing devices disposed about a data communications network 240.

In this regard, a multiplicity of surface sensor fixtures 290A, 290N are disposed about different locations of a bounded geographical area represented within a data structure 215 in the memory 220. Each of the surface sensor fixtures 290A, 290N may include an earthen sensor array 235A, an atmospheric sensor array 235B and a meteorological sensor array 235C. The earthen sensor array 235A includes different sensors adapted to measure soil conditions such as moisture or temperature. The atmospheric sensor array 235B includes different sensors adapted to measure atmospheric conditions such as the presence and fluxes of greenhouse gas and other gases, and the like. The meteorological sensor array 235C includes different sensors adapted to measure meteorological conditions such as wind speed and direction, radiation levels, barometric pressure, temperature and humidity. It bears noting as well that a cloud data service 280 accessible from over the data communications network 240 through remote server 270 provides meteorological and other environmental data both for locations in the bounded geographic area in which a surface sensor fixture 290A, 290N is present, and also for locations in which a surface sensor fixture 290A, 290N is not present.

Notably, a computing device 250 including a non-transitory computer readable storage medium can be included with the host computing platform 200 and accessed by the processing units 230 of one or more of the computers 210. The computing device 250 stores thereon or retains therein a program module 300 that includes computer program instructions which, when executed by one or more of the processing units 230, performs a programmatically executable process for spatial upscaling. Specifically, the program instructions during execution, at different time intervals, acquire observables for different locations in the bounded geographical area from the sensor arrays 235A, 235B, 235C of a surface sensor fixture 290A, 290N. Concurrently, the program instructions during execution acquire meteorological and other environmental information pertaining to the different locations in the bounded geographical area from cloud data service 280.

From the observables and meteorological and other environmental data from the sensor arrays 235A, 235B, 235C, a greenhouse gas flux measurement is computed for each corresponding one of the locations in which a disposed one of the surface sensor fixtures 290A, 290N is present. In this regard, the greenhouse gas flux is determined based upon a measurement of greenhouse gases by an associated one of the different surface sensor fixtures 290A, 290N at any given time, so that a directionality of movement of the greenhouse gas and quantity of the greenhouse gas being emitted into or removed from the atmosphere can be determined. The observables associated with measurements of soil and meteorological measurements act according to the known influence of such measurements on greenhouse gas fluxes and thus according to rule.

Importantly, the program instructions estimate the greenhouse gas flux of each location in the bounded geographic area in which a surface sensor fixture 290A, 290N is not disposed. Specifically, for each target location in the bounded geographic area in which one of the surface sensor fixtures 290A, 290N is not disposed, the program instructions query a flux model 225, previously trained to identify the flux values associated with different aspects of the environmental context within the geographically bounded area. The flux model 225 returns a predicted greenhouse gas flux for the target location using a weighted combination of measured fluxes and fluxes predicted by the model such that, for target locations closer to one of the surface sensor fixtures 290A, 290N the measured flux values from the sensor are most highly weighted whereas, as distance from one of the surface sensor fixtures increases, the weighting accorded to the measured flux values from that sensor assigned to the target location decreases, until the flux values become almost entirely predicted.

The flux model 225 may also compare the estimated fluxes with measured fluxes for locations in which one of the surface sensor fixtures 290A, 290N is disposed but whose flux measurements were not used to train the model, in which case the program instructions compute the differences between the estimated and measured fluxes and use the differences to correct the model estimates for unmonitored locations in which surface sensor fixtures 290A, 290N are not disposed. The program instructions then aggregate all greenhouse gas flux values for all of the locations of the bounded geographic area in order to arrive at a total greenhouse gas flux for the bounded geographic area. As such, the program instructions may mint an immutable token encapsulating the total greenhouse gas flux for storage in fixed storage 205.

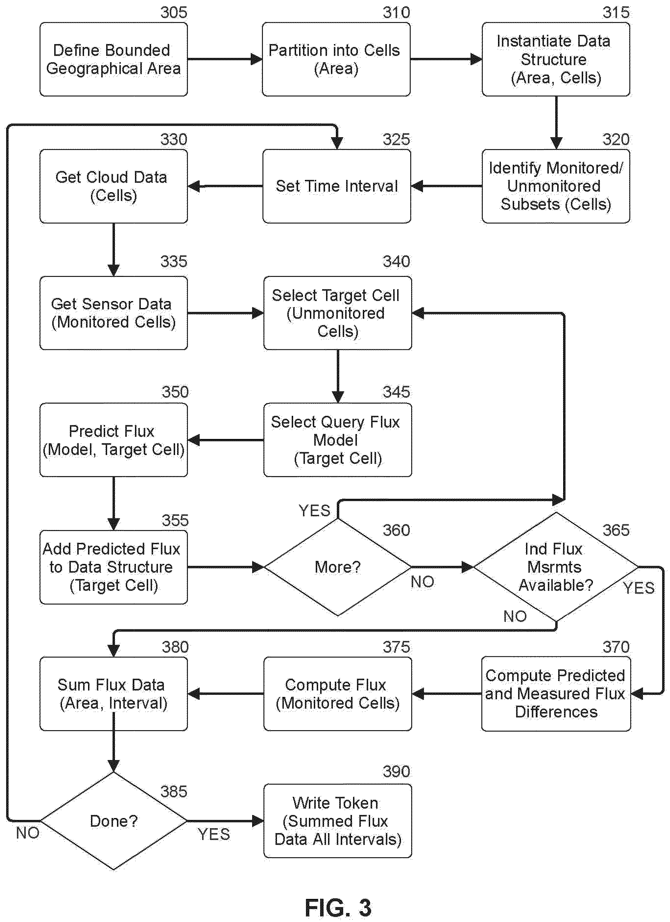

In further illustration of an exemplary operation of the module, Figure 3 is a flow chart illustrating one of the aspects of the process of Figure 1. Beginning in block 305, a bounded geographic area is defined and in block 310, the area is partitioned into cells. In block 315, a data structure - such as a multi-dimensional array or a table of records - is instantiated in memory to include a data object representative of each of the cells in the partitioned area. In block 320, each of the cells is classified in the data structure as either monitored by a surface sensor fixture disposed therein, or unmonitored in consequence of either: a) a lack of a surface sensor fixture disposed therein or because, b) although there is a surface sensor fixture disposed therein, the monitored cell is one of a selection of monitored cells whose data were not used previously in the training of the flux model which are therefore identified for that reason as unmonitored cells in block 320 so that the data from their surface sensor fixtures can be used for an independent check on the results of the upscaling process.

In block 325, a time interval is established during which a greenhouse gas flux is to be determined for the bounded geographic area. Then, in block 330 meteorological and other environmental data are retrieved from a cloud service providing such data for the different locations corresponding to the different cells in the bounded geographic area. As well, in block 335 fluxes are measured for the different monitored ones of the cells derived from sensor data retrieved from these cells' different surface sensor fixtures. In block 340, a first unmonitored one of the cells is selected as a target cell for spatial upscaling. Then, in block 345, a flux model, previously trained to identify the flux values associated with different aspects of the environmental context within the geographically bounded areas, is queried with meteorological and other environmental data for the target cell. The flux model, in response, provides a predicted greenhouse gas flux 350 for the unmonitored one of the cells. Thereafter, the predicted greenhouse gas flux is added to the data structure for the time interval in connection with the unmonitored one of the cells. In decision block 360, if additional unmonitored ones of the cells remain to be processed for spatial upscaling, the process returns to block 340 with the selection of a new target one of the unmonitored cells until no further unmonitored ones of the cells remain to be processed.

At that point, another decision, in block 365, is made based on whether a selection of monitored cells is available whose data were not used previously in the training of the flux model 345 and which were therefore identified for that reason as unmonitored cells in block 320. If so, in block 370 the process computes the differences between the flux measurements for the selection of monitored cells whose data were not used previously in the training of the flux model 345, and which were therefore identified for that reason as unmonitored cells in block 320, and the flux values predicted by the flux model 345 for those cells and assigned to those cells in block 350.

These differences are then used in block 375 to correct, as necessary, the predicted flux values assigned to all of the different ones of the unmonitored cells in block 350. In block 380, the measured and predicted greenhouse gas fluxes for all of the different cells are then summed as the greenhouse gas flux for the bounded geographic area for the time interval. In decision block 385, if the greenhouse gas flux is to be computed for an additional time interval, the process returns to block 325 with the establishment of the new time interval. But otherwise, in block 390 an immutable token is created to encapsulate the greenhouse gas flux for any or all of the time intervals previously processed.

Of import, the foregoing flowchart and block diagram referred to herein illustrate the architecture, functionality, and operation of possible implementations of systems, methods, and computing devices according to various embodiments of the present invention. In this regard, each block in the flowchart or block diagrams may represent a module, segment, or portion of instructions, which includes one or more executable instructions for implementing the specified logical function or functions. In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

More specifically, the present invention may be embodied as a programmatically executable process. As well, the present invention may be embodied within a computing device upon which programmatic instructions are stored and from which the programmatic instructions are enabled to be loaded into memory of a data processing system and executed therefrom in order to perform the foregoing programmatically executable process. Even further, the present invention may be embodied within a data processing system adapted to load the programmatic instructions from a computing device and to then execute the programmatic instructions in order to perform the foregoing programmatically executable process.

To that end, the computing device is a non-transitory computer readable storage medium or media retaining therein or storing thereon computer readable program instructions. These instructions, when executed from memory by one or more processing units of a data processing system, cause the processing units to perform different programmatic processes exemplary of different aspects of the programmatically executable process. In this regard, the processing units each include an instruction execution device such as a central processing unit or "CPU" of a computer. One or more computers may be included within the data processing system. Of note, while the CPU can be a single core CPU, it will be understood that multiple CPU cores can operate within the CPU and in either instance, the instructions are directly loaded from memory into one or more of the cores of one or more of the CPUs for execution.

Aside from the direct loading of the instructions from memory for execution by one or more cores of a CPU or multiple CPUs, the computer readable program instructions described herein alternatively can be retrieved from over a computer communications network into the memory of a computer of the data processing system for execution therein. As well, only a portion of the program instructions may be retrieved into the memory from over the computer communications network, while other portions may be loaded from persistent storage of the computer. Even further, only a portion of the program instructions may execute by one or more processing cores of one or more CPUs of one of the computers of the data processing system, while other portions may cooperatively execute within a different computer of the data processing system that is either co-located with the computer or positioned remotely from the computer over the computer communications network with results of the computing by both computers shared therebetween.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

Having thus described the invention of the present application in detail and by reference to embodiments thereof, it will be apparent that modifications and variations are possible without departing from the scope of the invention defined in the appended claims as follows:

## Claims

1. A spatial upscaling method for greenhouse gas flux quantification over a sparsely sensed geographically bounded region, the method comprising:
defining a geographically bounded area in a data structure in memory of a host computing platform;
configuring the data structure to include a discrete number of uniform spatial units, each of the units representative of a specific location within the geographically bounded area;
receiving greenhouse gas flux data from different sensors disposed within the geographically bounded area and storing the flux data in one or more of the corresponding uniform spatial units of the data structure;
receiving environmental context data for each location within the geographically bounded area and storing them in the corresponding one of the uniform spatial units of the data structure;
for each one of the uniform spatial units in the data structure not associated with a disposed one of the different sensors:
retrieving environmental context data for the uniform spatial unit not associated with a disposed one of the sensors;
querying a flux model with the retrieved environmental context data to obtain predictive data for gas flux at a location in the geographically bounded area corresponding to the uniform spatial unit in the data structure not associated with a disposed one of the sensors, the flux model estimating a flux value for locations not associated with a disposed one of the sensors within the geographically bounded area reflective of the environmental context data and known flux values for comparable known locations within the geographically bounded area; and,
assigning the predictive data for gas flux to a portion of the data structure associated with the corresponding one of the uniform spatial units not associated with a disposed one of the sensors; and,
generating an emissions or removals token from the data structure indicative of a total mass of greenhouse gas emitted or sequestered across all locations within the geographically bounded area.

2. The method of claim 1, further comprising discarding any portion of the gas flux data computed to fall outside a threshold range relative to a running average of gas flux data for each disposed sensor.

3. The method of claim 1, wherein the sensors each include a greenhouse gas flux sensor measuring a concentration of greenhouse gas in atmosphere and a directionality of flow of the greenhouse gas.

4. The method of claim 1, wherein the environmental context data include at least one of humidity, temperature, wind speed and direction and vegetation.

5. A data processing system adapted for greenhouse gas flux quantification over a sparsely sensed geographically bounded region, the system comprising:
a host computing platform comprising one or more computers, each with memory and one or processing units including one or more processing cores;
a multiplicity of different sensors disposed within a geographically bounded area, each communicatively coupled to a network interface of the host computing platform;
a data structure stored in the memory and defining the geographically bounded area; and,
a spatial upscaling module comprising computer program instructions enabled while executing in the memory of at least one of the processing units of the host computing platform to perform:
configuring the data structure to include a discrete number of uniform spatial units, each of the units representative of a specific location within the geographically bounded area;
receiving greenhouse gas flux data from the different sensors and storing the flux data in one or more of the corresponding uniform spatial units of the data structure;
receiving environmental context data for each location within the geographically bounded area and storing them in the corresponding one of the uniform spatial units of the data structure;
for each one of the uniform spatial units in the data structure not associated with a disposed one of the different sensors:
retrieving environmental context data for the uniform spatial unit not associated with a disposed one of the sensors;
querying a flux model with the retrieved environmental context data to obtain predictive data for gas flux at a location in the geographically bounded area corresponding to the uniform spatial unit in the data structure not associated with a disposed one of the sensors, the flux model estimating a flux value for locations not associated with a disposed one of the sensors within the geographically bounded area reflective of the environmental context data and known flux values for comparable known locations within the geographically bounded area; and,
assigning the predictive data for gas flux to a portion of the data structure associated with the corresponding one of the uniform spatial units not associated with a disposed one of the sensors; and,
generating an emissions or removals token from the data structure indicative of a total mass of greenhouse gas emitted or sequestered across all locations within the geographically bounded area.

6. The system of claim 5, wherein the program instructions further perform discarding any portion of the gas flux data computed to fall outside a threshold range relative to a running average of gas flux data for each disposed sensor.

7. The system of claim 5, wherein the sensors each include a greenhouse gas flux sensor measuring a concentration of greenhouse gas in atmosphere and a directionality of flow of the greenhouse gas.

8. The system of claim 5, wherein the environmental context data include at least one of humidity, temperature, wind speed and direction and vegetation.

9. A computing device comprising a non-transitory computer readable storage medium having program instructions stored therein, the instructions being executable by at least one processing core of a processing unit to cause the processing unit to perform a spatial upscaling for greenhouse gas flux quantification over a sparsely sensed geographically bounded region by:
defining a geographically bounded area in a data structure in memory of a host computing platform;
configuring the data structure to include a discrete number of uniform spatial units, each of the units representative of a specific location within the geographically bounded area;
receiving greenhouse gas flux data from different sensors disposed within the geographically bounded area and storing the flux data in one or more of the corresponding uniform spatial units of the data structure;
receiving environmental context data for each location within the geographically bounded area and storing them in the corresponding one of the uniform spatial units of the data structure;
for each one of the uniform spatial units in the data structure not associated with a disposed one of the different sensors:
retrieving environmental context data for the uniform spatial unit not associated with a disposed one of the sensors;
querying a flux model with the retrieved environmental context data to obtain predictive data for gas flux at a location in the geographically bounded area corresponding to the uniform spatial unit in the data structure not associated with a disposed one of the sensors, the flux model estimating a flux value for locations not associated with a disposed one of the sensors within the geographically bounded area reflective of the environmental context data and known flux values for comparable known locations within the geographically bounded area; and,
assigning the predictive data for gas flux to a portion of the data structure associated with the corresponding one of the uniform spatial units not associated with a disposed one of the sensors; and,
generating an emissions or removals token from the data structure indicative of a total mass of greenhouse gas emitted or sequestered across all locations within the geographically bounded area.

10. The device of claim 9, wherein the instructions further perform spatial upscaling by additionally discarding any portion of the gas flux data computed to fall outside a threshold range relative to a running average of gas flux data for each disposed sensor.

11. The device of claim 9, wherein the sensors each include a greenhouse gas flux sensor measuring a concentration of greenhouse gas in atmosphere and a directionality of flow of the greenhouse gas.

12. The device of claim 9, wherein the environmental context data includes at least one of humidity, temperature, wind speed and direction and vegetation characteristics.
